# FASCICULE DE BREVET EUROPEEN

(11) **EP 2 307 064 B1**
(45) Date de publication et mention de la délivrance du brevet: **14.03.2018**
(21) Numéro de dépôt: 09797556.9
(22) Date de dépôt: 22.06.2009
(51) Int. Cl.: A61L 27/12, A61L 27/54

(54) **BIOMATERIAUX A BASE DE PHOSPHATE DE CALCIUM**
CALCIUMPHOSPHATHALTIGE BIOMATERIALIEN
BIOMATERIALS CONTAINING CALCIUM PHOSPHATE

(30) Priorité: 23.06.2008 FR 0803493
(43) Date de publication de la demande: 13.04.2011
(73) Titulaire: Centre National de la Recherche Scientifique, 75016 Paris (FR); CENTRE HOSPITALIER UNIVERSITAIRE DE NICE, 06003 Nice Cedex 1 (FR)
(72) Inventeur: BALAGUER, Thierry, F-06670 Colomars (FR); ROCHET, Nathalie, F-06100 Nice (FR); CARLE, Georges, F-06000 Nice (FR)
(74) Mandataire: Corizzi, Valérie
(86) Numéro de dépôt international: PCT/FR2009/000748
(87) Numéro de publication internationale: WO 2010/007229

(56) Documents cités:
- WO-A-2006/015275
- WO-A-2006/058153
- WO-A-2008/104762
- US-A1- 2005 226 939
- M. H. MANKANI ET AL.: "In vivo Bone Formation by Human Bone Marrow Stromal Cells: Effect of Carrier Particle Size and Shape" BIOTECHNOLOGY AND BIOENGINEERING, vol. 72, no. 1, 5 janvier 2001 (2001-01-05), pages 96-107, XP002514537 cité dans la demande
- HERTZ A ET AL: "Inorganic materials for bone repair or replacement applications" NANOMEDICINE, FUTURE MEDICINE LTD., LONDON, GB, vol. 2, no. 6, 1 décembre 2007 (2007-12-01), pages 899-918, XP008102023 ISSN: 1743-5889

## Description

L'invention a pour objet un nouveau biomatériau à base de phosphate de calcium, notamment à base d'hydroxyapatite ou à base d'un matériau comprenant de l'hydroxyapatite tel que les phosphates de calcium biphasés et les ciments phosphocalciques, un procédé pour sa préparation et son utilisation pour la fabrication d'un implant ou pour la pose d'une prothèse pour permettre la régénération du tissu osseux.

La reconstruction des pertes de substance osseuse, d'origine principalement traumatique et plus rarement tumorale, est une des grandes difficultés rencontrées par les chirurgiens orthopédistes. Les défauts de petite taille, depuis la pseudarthrose « serrée » (défaut de consolidation d'une fracture où la perte de substance est virtuelle) jusqu'à des pertes osseuses de 5-6 cm font, le plus souvent, l'objet d'une greffe autologue de tissu osseux spongieux ou cortico-spongieux prélevé sur la crête iliaque (gold standard). Les défauts de grandes tailles (≥ 6 cm) nécessitent des interventions beaucoup plus lourdes, transferts osseux vascularisés ou procédure de Masquelet. Malgré tout, la quantité d'os autologue disponible est limitée, la consolidation osseuse reste aléatoire et ces différentes techniques sont fortement pourvoyeuses de complications post opératoires au niveau du site de prélèvement de la greffe.

Différents biomatériaux disponibles en pratique clinique permettent d'éviter, en théorie, les inconvénients de la greffe autologue. Malheureusement, aucun d'eux n'égale les résultats de la greffe osseuse et ils ne permettent jamais la reconstruction de pertes de substance de grande taille.

La majorité des matériaux étudiés actuellement associent aux biomatériaux des cellules souches mésenchymateuses obtenues à partir de moelle osseuse après plusieurs semaines de sélection et de culture cellulaire *in vitro.* Cette approche est lourde et coûteuse, ce qui limite les retombées cliniques.

Il est connu que le sang coagulé favorise la reconstruction osseuse. L. Okazaki et al., Clin. Oral Impl. Res., 16, 2005, 236-243 décrit des implants à base de poudre d'os déminéralisé ou de sang coagulé. Le document WO 02/068010 décrit un matériau composite à base de moelle osseuse, ce matériau comprenant une matrice implantable biocompatible et poreuse et un matériau coagulé, tel qu'un coagulat de moelle osseuse, de sang, de plasma.

De tels matériaux, résultant de l'association d'un support et de sang coagulé ou non, ont été jusqu'à présent utilisés en chirurgie maxillo-faciale où les problèmes de consolidation osseuse sont peu importants, mais ils ont été peu ou pas utilisés dans la réparation d'os diaphysaires.

Les procédés de fabrication de ces implants nécessitent de prélever du sang sur un donneur, le plus souvent le destinataire de l'implant, et requièrent ensuite des étapes de manipulation du support (os déminéralisé ou polymère de synthèse, céramique), notamment des étapes de mélange avec le sang, qui sont sources de contamination du biomatériau. En outre, il est difficile par ces procédés d'obtenir un biomatériau homogène.

Il subsiste donc le besoin d'un procédé de préparation d'un biomatériau implantable à partir d'un support de synthèse, donc facile à produire, avec des propriétés constantes et homogènes, ce procédé permettant d'obtenir des propriétés supérieures en termes de biocompatibilité, et permettant la reconstruction rapide d'un tissu osseux de qualité, sans qu'il soit nécessaire de faire appel à des étapes de culture ou de prélèvement.

L'invention permet de remédier aux inconvénients de l'art antérieur et permet l'obtention d'un os d'excellente qualité en termes de dureté et de vascularisation. En outre le procédé de fabrication de ce biomatériau est simple, facile à mettre en oeuvre, ne nécessite pas de multiples interventions sur l'individu à traiter, est peu coûteux comparativement aux procédés de l'art antérieur.

L'hydroxyapatite entre dans la composition de nombreux matériaux de reconstruction osseuse. L'hydroxyapatite peut être employée seule dans cette application ou en mélange avec d'autres composants, comme c'est le cas par exemple dans le phosphate de calcium biphasique, ou dans les ciments phosphocalciques.

Le phosphate de calcium biphasique, BCP, est utilisé dans de nombreuses applications médicales et dentaires. Le phosphate de calcium biphasique a été décrit pour la première fois comme matériau de réparation osseuse par Nery et al., J Periodontol. 1992 Sep; 63(9):729-35. Le BCP est constitué d'un mélange d'hydroxyapatite (HA) Ca₁₀(PO₄)₆(OH)₂ et de phosphate tricalcique bêta (Ca₃(PO₄)₂) (β-TCP). Sa bioactivité et sa biorésorbatilité peuvent être contrôlées par la proportion d'hydroxyapatite et de β-TCP qui le constituent.

Le document US-2005/0226939 décrit un procédé de fabrication de nanoparticules d'hydroxyapatite, ce procédé comportant le mélange d'une composition à base d'ions calcium et d'ions phosphate et un traitement par des micro-ondes. Les conditions employées dans ce document ne conduisent pas à la formation d'hydroxyapatite ou de BCP imprégné par une solution de chlorure de calcium.

Les biomatériaux à base de BCP ont l'avantage, par rapport aux autres biomatériaux de synthèse, de favoriser l'ostéogenèse.

Le BCP a fait l'objet de nombreuses études : Lerouxel et al. Biomaterials, 2006, Sept. 27(26) :4566-72, 18, 287-294 ; Malard O. et al., J. Biomed. Mater. Res., 46(1), 1999, 103 ; Mankani M.H. et al., Biotechnology and Bioengineering, 72(1), 2001, 96-107. Ces différents auteurs ont produit des observations relatives à l'influence de la taille des particules de BCP. Dans le document Mankani M.H. *et al.,* le procédé comporte le mélange de particules de HA/TCP avec des cellules puis du fibrinogène, et de la thrombine reconstituée dans une solution de CaCl₂. Mais la solution de CaCl₂ est plus concentrée que celle utilisée dans l'invention et le ratio mole/poids de CaCl₂/ BCP est supérieur à celui employé dans les biomatériaux de l'invention.

Trojani C. et al., Biomaterials, 27, 2006, 3256-3264, ont montré qu'une bonne ostéoinduction pouvait être obtenue pour l'implantation d'un matériau composite BCP/hydrogel de Si- hydroxypropylméthyl cellulose auquel ont été additionnées des cellules de moelle osseuse, avec des particules de BCP calibrées de 40 à 80 µm. Ces derniers procédés requièrent cependant une étape de prélèvement de cellules de moelle osseuse ainsi que leur mise en culture.

Le document WO 2006/015275 décrit un procédé favorisant la régénération osseuse, ce procédé comprenant la préparation d'une composition constituée d'un matériau support à base de phosphate de calcium, d'un plasma riche en plaquettes, de calcium et un activateur du récepteur PAR distinct de la thrombine. Mais la concentration en CaCl₂ dans ces compositions est si élevée qu'il agirait, s'il était mis en oeuvre dans les conditions de la présente invention, comme anticoagulant.

Les deux composants du BCP, HA et β-TCP, représentent les deux principaux types de phosphates de calcium utilisés en chirurgie osseuse et dentaire. Ils sont remarquablement biocompatibles et on considère que leur association présente une meilleure bioactivité et donc une plus grande efficacité que l'HA seule ou le β-TCP seul. En effet, dans les BCP les deux composantes (HA et β-TCP) présentent une synergie d'action :

L'hydroxyapatite, dès son implantation *in vivo* et de par sa structure chimique, peut promouvoir à sa surface, la formation d'apatite phosphocalcique non stoechiométrique polysusbtituée (dites « apatites biologiques ») par croissance épitaxique. Cette couche d'apatite biologique, très proche des cristaux présents dans la matrice osseuse, faciliterait l'adhésion et l'activité cellulaires.

Le β-TCP, beaucoup plus soluble que l'HA, maintient une sursaturation en calcium et phosphates des fluides biologiques environnant l'implant en BCP. Ceci permet d'entretenir le phénomène de précipitation d'apatite biologique sur la phase HA. De plus cette phase est beaucoup plus résorbable que HA, il est donc possible de moduler la résorbabilité de l'implant en faisant varier le rapport HA/β-TCP.

Ces phénomènes chimiques ont été montrés *in vitro* (J.M. Bouler, G. Daculsi, Key Engineering Materials 2001 ;192-195:119-122; Yamada S, et al., Biomaterials 1997;18:1037-41 ; S. Yamada et al., J. Biomed. Mater. Res 1997;37:346-52) et in vivo (Daculsi G, et al., J Biomed Mater Res 1989;23:883-94 ; G. Daculsi et al. Int. Rev. Cytol 1997;129-191). Ces mécanismes semblent participer à la meilleure efficacité clinique des BCP par rapport aux matériaux monophasés en HA ou TCP (Nery EB et al., J Periodontol 1992;63:729-35 ; Ellinger RFet al., Int J Periodontics Restorative Dent 1986;6:22-33 ; Passuti N. et al., Clin Orthop Relat Res 1989;(248):169-76 ; Gouin F et al., Rev Chir Orthop Reparatrice Appar Mot 1995;81:59-65 ; Ransford AOet al., J Bone Joint Surg Br 1998;80:13-8 ; R. Cavagna et al., J. Long-Term effect of Med. Impl 1999;9:403-412).

La présente invention se fonde sur la constatation que certains phosphates de calcium, en particulier les apatites phosphocalciques, comme l'hydroxyapatite, inhibent la coagulation spontanée du sang total lorsqu'ils sont mis en contact. En effet, il a été constaté que l'HA, et aussi le BCP, qui contient de l'HA, inhibent la coagulation spontanée du sang total lorsqu'ils sont mis en contact. Il a été constaté aussi que si l'on imprègne au préalable l'hydroxyapatite ou le BCP avec du sérum physiologique (qui est une phase aqueuse de NaCl), comme recommandé dans les notices d'utilisation de ces biomatériaux, le sang mis en contact ensuite ne coagule pas.

Des conditions expérimentales permettant d'établir les propriétés anticoagulantes d'un matériau support sont détaillées dans la partie expérimentale.

L'invention a pour premier objet un procédé de préparation d'un biomatériau implantable comportant un support à base d'au moins un phosphate de calcium tel que l'hydroxyapatite ou un mélange d'hydroxyapatite et d'au moins un autre matériau, ce procédé comportant au moins une étape d'imprégnation du support par au moins un agent coagulant.

Par support à base de phosphates de calcium on entend un matériau comprenant au moins un constituant du type apatite phosphocalcique choisi parmi : l'hydroxyapatite, la fluorapatite, les apatites non stoechiométriques polysubstituées (ANSPS) ainsi que leurs mélanges avec d'autres biomatériaux phosphocalciques. De tels supports peuvent être constitués par de l'hydroxyapatite, du BCP, des ANSPS et des ciments phosphocalciques apatitiques.

Le support ainsi imprégné est ensuite implanté sur le site où un défaut osseux doit être comblé. Son implantation provoque alors la coagulation du sang qui entre en contact avec le biomatériau ou qui pénètre dans le biomatériau *in situ.* Dans les essais qui ont été réalisés avec du BCP, il a été constaté que le BCP, associé à du sang coagulé, permet d'obtenir une bonne ostéogenèse et conduit à un tissu osseux de qualité très satisfaisante par un procédé très simple comparativement à ceux de l'art antérieur. Sans imprégnation du BCP par une solution d'agent coagulant, on n'obtient pas d'ostéogenèse.

Selon une variante de l'invention, le support à base de phosphate de calcium, notamment à base d'hydroxyapatite ou d'un mélange d'hydroxyapatite et d'un autre matériau est implanté dans le site où un défaut osseux doit être comblé puis il est imprégné *in situ* par une solution d'agent coagulant.

De préférence, le support utilisé dans l'invention est à base d'hydroxyapatite, de fluorapatite, d'apatites non stoechiométriques polysubstituées (ANSPS) ou d'un mélange d'un de ces composés avec au moins un autre biomatériau tel que le phosphate tricalcique sous forme α et β (Ca₃(PO₄)₂), le phosphate dicalcique dihydraté (CaHPO₄, 2H₂O), le phosphate dicalcique anhydre (CaHPO₄), le phosphate monocalcique monohydrate (Ca (HPO₄)₂, H₂0), le phosphate tétracalcique (Ca (PO₄)₂ O), et le phosphate octocalcique (Ca₈ H₂ (PO₄)₆). De façon avantageuse, le support est à base d'hydroxyapatite ou de BCP, de préférence il est à base de BCP.

Le support, et notamment l'apatite ou le BCP qui peuvent être utilisés dans l'invention peuvent être de toute forme : soit sous la forme d'un monolithe soit sous forme de granulés, calibrés ou non.

Le BCP qui peut être utilisé dans l'invention consiste en un fritté haute température. Lorsqu'il est sous forme de granulés il est broyé et calibré, par exemple par tamisage, en fonction du diamètre choisi. De façon avantageuse le BCP qui peut être utilisé dans l'invention comporte de l'hydroxyapatite et du phosphate tri calcique β dans un rapport en poids/poids HA/ β-TCP compris entre 5/95 et 95/5, de préférence entre 30/70 et 80/20, avantageusement entre 40/60 et 60/40.

Avantageusement il s'agit d'un support, et en particulier d'un BCP, poreux, avec des tailles de pores allant de 50 nm à 1000 µm, avantageusement de 500 nm à 100 µm et plus avantageusement de 1 µm à 50 µm.

Lorsque le support, et en particulier le BCP, utilisé dans la présente invention est sous forme de granulés, il présente avantageusement une granulométrie comprise entre 40 et 500 µm, préférentiellement entre 40 et 400 µm, encore plus préférentiellement entre 40 et 300 µm, et avantageusement entre 80 et 200 µm.

Les granulés ou la poudre de BCP peuvent être obtenus conformément aux méthodes décrites par Bouler et al., J Biomed Mater Res, 1996, 32, 603-609 ; Bouler et al., J Biomed Mater Res, 2000, 51, 680-684 ; Obadia et al., J Biomed Mater Res, 2006, 80(B), 32-42.

Le BCP peut être obtenu commercialement auprès de la société GRAFTYS SARL (Aix en Provence).

L'hydroxyapatite qui peut être utilisée dans l'invention est préférentiellement sous la forme de granulés. Elle est disponible commercialement auprès de la société GRAFTYS SARL.

Plus particulièrement, l'invention concerne un biomatériau comprenant un support à base de phosphate de calcium, imprégné par une solution d'au moins un agent coagulant dérivé du calcium, le support étant choisi parmi : de l'hydroxyapatite et un BCP, l'agent coagulant est sous forme d'une solution aqueuse d'une concentration allant de 1 à 50 mMol/l, la proportion de solution d'agent coagulant et de HA ou de BCP est de 0,5 à 5 en volume/volume de solution d'agent coagulant par rapport au volume de HA ou de BCP.

De façon préférentielle, l'invention concerne un biomatériau comprenant un support à base de phosphate de calcium, imprégné par une solution d'au moins un agent coagulant dérivé du calcium, le support étant choisi parmi : de l'hydroxyapatite et un BCP, l'agent coagulant dérivé du calcium étant présent en proportion allant de 2,5 à 60 µmol de calcium par gramme de HA ou de BCP, et de préférence de 5 à 40 µmol de calcium par gramme de HA ou de BCP.

L'agent coagulant est un agent coagulant à base de calcium, tel qu'un sel de calcium biocompatible, comme par exemple : CaCl₂, Ca(NO₃)₂, Ca(AcOEt)₂, CaSO₄.

Avantageusement l'agent coagulant est à base de calcium et il est choisi parmi les sels de calcium biocompatibles, avantageusement CaCl₂. Pour permettre l'imprégnation du support, en particulier du HA ou du BCP, par l'agent coagulant, ce dernier est utilisé en solution aqueuse, avantageusement en solution aqueuse d'une concentration allant de 1 à 50 mMol/l, de préférence de 3 à 40 mMol/l, avantageusement de 5 à 20 mMol/l. Ces valeurs sont particulièrement préférées dans le cas où l'agent coagulant est un sel de calcium et notamment CaCl₂.

La proportion de solution d'agent coagulant et de HA ou de BCP mis en oeuvre dans le procédé de l'invention est de 0,5 à 5 en volume/volume de solution d'agent coagulant par rapport au poids de HA ou de BCP, de préférence de 1 à 3, avantageusement environ 2.

Selon une autre variante de l'invention, le biomatériau est préparé de façon extemporanée, par imprégnation du support à base de phosphate de calcium juste avant son implantation.

On peut aussi prévoir de préparer le biomatériau de l'invention en appliquant la procédure suivante : imprégner le support à base de phosphates de calcium par une solution d'agent coagulant puis le sécher ou le lyophiliser puis le conditionner dans des conditions stériles et le conserver jusqu'à son implantation.

Avantageusement, la durée de l'imprégnation est de 1 minute à 1 heure, de préférence de 1 à 30 minutes, avantageusement de 5 à 15 minutes.

Selon une autre variante de l'invention, on peut préparer un biomatériau de l'invention en imprégnant le support à base de phosphate de calcium par une solution d'agent coagulant puis on conditionne ce biomatériau dans des conditions stériles et on le conserve ainsi jusqu'à son implantation.

Selon une variante de l'invention, on peut prévoir que le biomatériau support (à base de phosphate de calcium) est associé à l'agent coagulant sous forme de poudre. En particulier on peut mélanger un biomatériau tel que du BCP ou de l'HA sous forme de poudre ou de granulés avec un sel de calcium sous forme de poudre solide. Ce biomatériau peut ainsi être stocké jusqu'à son utilisation et imprégné par une solution aqueuse, comme par exemple du sérum physiologique, de façon extemporanée, juste avant son implantation, au moment de son utilisation. Il peut aussi être implanté sous forme sèche, non imprégnée.

Selon l'invention, on peut prévoir qu'au support, et notamment au BCP, soient ajoutés un ou plusieurs additifs éventuels tels que : polymères, particules de céramiques, molécules pharmaceutiques, agents bioactifs, les conditions pour l'emploi de ces matériaux étant : leur biocompatibilité, l'absence d'effet négatif sur la réaction de coagulation sanguine. Si l'un de ces additifs avait un effet défavorable sur la coagulation sanguine, celui-ci devrait être pris en compte dans la quantité d'agent coagulant à mettre en oeuvre. Par exemple, de tels additifs ou actifs peuvent être employés par greffage du support, BCP ou autre, par mélange ou imprégnation ou par enrobage. De tels additifs bien connus de l'homme du métier sont destinés à modifier la rhéologie du biomatériau, son comportement *in vivo* (dureté, résorption, ostéogenèse) ou à agir sur l'apparition d'infections ou de phénomènes inflammatoires (antibiotiques, anti-infectieux, agents anti-inflammatoires).

On peut aussi prévoir d'introduire dans le biomatériau de l'invention une ou plusieurs molécules thérapeutiques comme des molécules destinées à prévenir ou traiter une pathologie choisie par exemple parmi : un cancer, l'ostéoporose.

On peut aussi prévoir d'introduire dans le biomatériau de l'invention du tissu adipeux, ou toute autre préparation de tissu ou de cellules, prélevé chez le patient auquel est destiné le biomatériau, ce tissu adipeux ou cette préparation ayant été préalablement mis en suspension dans du sang ou du plasma ou du sérum physiologique.

On peut également introduire dans le biomatériau de l'invention des facteurs de croissance, naturels ou de synthèse. On peut aussi prévoir la présence de biomarqueurs ou d'agents de contraste qui favorisent la visualisation par imagerie médicale de la résorption du biomatériau et son devenir dans l'organisme.

Selon le procédé de l'invention, le support, et notamment le HA ou le BCP, est placé dans une cavité d'un récipient fermé et stérile. Lorsque le support est sous forme de granulés, on peut par exemple le placer dans la cavité intérieure d'une seringue. La quantité appropriée d'agent coagulant est introduite dans ce récipient, par exemple par aspiration à l'aide de la seringue si l'on a utilisé un tel dispositif.

Dans le cas où le support, et notamment le HA ou le BCP, est sous forme d'un monolithe, celui-ci est placé dans un récipient de forme et de dimensions appropriées.

Dans tous les cas le volume du récipient est prévu pour permettre l'introduction de la quantité voulue de solution d'agent coagulant.

Le récipient clos contenant le support, notamment le HA ou le BCP, et l'agent coagulant peut être agité, de façon à permettre l'imprégnation homogène du biomatériau. Mais on peut aussi prévoir une imprégnation passive du support par l'agent coagulant.

A l'issue de cette étape, le biomatériau est sous forme :
- d'une pâte liquide, homogène, lorsque le support a été employé sous forme de granulés,
- d'un monolithe dont les cavités sont remplies de liquide, lorsque le support a été employé sous forme d'un monolithe.

Selon une variante de l'invention on peut prévoir d'implanter directement le matériau support dans l'espace à combler, éventuellement en mélange avec l'agent coagulant sous forme de poudre, puis de l'imprégner *in situ*, soit par la solution d'agent coagulant, soit lorsqu'il est déjà en mélange avec l'agent coagulant, avec une solution aqueuse adaptée telle que du sérum physiologique. On peut aussi prévoir de l'implanter, lorsqu'il est sous forme de mélange sec avec l'agent coagulant, sans l'imprégner, et de le laisser être imprégné par le sang des tissus environnants.

Un autre objet de l'invention est un biomatériau comportant un support à base de phosphate de calcium tel que l'hydroxyapatite ou un BCP, imprégné par une solution d'au moins un agent coagulant, tel que décrit ci-dessus.

Suivant la forme physique du support, HA ou BCP, et le type de dispositif qui a été employé pour la préparation du biomatériau de l'invention, celui-ci peut ensuite être appliqué à l'aide des moyens les plus adaptés à l'emplacement où un défaut osseux doit être comblé :

A l'aide d'un outil tel qu'une spatule, ou à l'aide d'une seringue dont l'extrémité comporte une ouverture adaptée à la rhéologie et à la taille des particules du biomatériau de l'invention. Il peut aussi être implanté directement sous forme d'un monolithe. Dans ce dernier cas celui-ci aura été conçu ou taillé pour que sa forme et ses dimensions correspondent à celles de l'espace à combler.

L'invention a encore pour objet un procédé de comblement d'un défaut osseux, ce procédé comportant les étapes énumérées ci-dessus et comportant en outre une étape d'insertion du biomatériau dans l'espace où un défaut osseux a été constaté. Ce procédé peut en outre comprendre des étapes d'incision de tissu et de suture.

Suivant la taille et la configuration du défaut osseux, le comblement par le biomatériau de l'invention peut être associé à la pose d'une ostéosynthèse provisoire qui permet de conférer au tissu atteint la résistance mécanique nécessaire pendant que se produit la reconstruction osseuse sur le site d'implantation du biomatériau de l'invention.

Comme les inventeurs l'ont constaté, l'implantation du biomatériau de l'invention a permis de favoriser la formation de tissu osseux dans des délais courts (quelques semaines), ce tissu osseux étant très richement vascularisé.

Un autre objet de l'invention est constitué par un kit pour la mise en oeuvre du procédé de l'invention, ce kit comprenant l'association d'un support à base d'un phosphate de calcium comme une hydroxyapatite ou un mélange d'hydroxyapatite et d'au moins un autre matériau, comme par exemple un BCP microporeux, avec un agent coagulant dérivé du calcium. Avantageusement l'agent coagulant est un sel de calcium biocompatible, comme CaCl₂.

La quantité d'agent coagulant est calculée pour compenser l'effet anticoagulant du phosphate de calcium, et en particulier de l'hydroxyapatite et favoriser la coagulation sanguine des tissus environnants.

Une telle association peut être sous la forme d'un kit comportant :
(a) un dispositif stérile comportant une cavité intérieure stérile dans lequel est placé le support, comme par exemple le BCP ou l'HA,
(b) un réservoir stérile comportant l'agent coagulant.

Le réservoir (b) peut faire partie du dispositif (a) ou être une entité distincte telle qu'un tube ou un flacon dans lequel on peut prélever l'agent coagulant pour le transférer dans la cavité intérieure du dispositif (a), ou une seringue permettant l'injection de l'agent coagulant dans la cavité où est placé le support.

La cavité intérieure du dispositif (a) est d'une taille permettant d'y introduire la quantité d'agent coagulant nécessaire pour produire le biomatériau de l'invention, ainsi que les autres constituants du mélange tels que par exemple des principes actifs.

De façon avantageuse également le dispositif (a) comporte des moyens permettant l'application du biomatériau dans la zone où un défaut osseux a été constaté.

Un tel dispositif peut être constitué d'une seringue.

On peut également prévoir d'utiliser un dispositif tel que celui décrit dans WO 02/068010 comportant un tube à l'intérieur duquel est conservé le support, notamment le BCP, dans lequel on injecte l'agent coagulant et qui peut se voir adapter un piston pour restituer le biomatériau une fois que celui-ci est formé.

Un biomatériau selon l'invention peut être utilisé pour la fabrication d'un implant osseux, qu'il s'agisse de combler une fracture, une perte de substance d'origine traumatique ou tumorale, un défaut consécutif à une intervention chirurgicale, ou d'aider à la mise en place d'une prothèse.

Le biomatériau peut être introduit par une intervention chirurgicale dans la zone où un défaut osseux doit être comblé. Après incision, le biomatériau est implanté et l'incision est refermée.

Le biomatériau de l'invention peut être associé à une ostéosynthèse de façon à permettre la consolidation provisoire en attendant la stabilisation de la zone déficiente par les tissus osseux.

Un enrobage de la prothèse par le biomatériau de l'invention permet de favoriser l'implantation de tissu osseux vivant dans ou autour de la prothèse.

Le biomatériau de l'invention peut encore être utilisé *in vitro* ou *ex*-*vivo* comme support pour la production de tissu osseux :

En effet, la culture de cellules osseuses autour de ce biomatériau permet de produire un tissu osseux ultérieurement implantable.

Un autre objet de l'invention est l'utilisation *in vitro* ou *ex-vivo* d'un biomatériau tel que décrit ci-dessus pour produire un implant osseux.

On peut, selon l'invention cultiver des cellules osseuses sur le biomatériau de l'invention dans un moule ayant la forme de la prothèse que l'on souhaite fabriquer. La culture de cellules dans ces conditions permet d'obtenir une prothèse biocompatible de forme et de dimensions adaptées.

### FIGURES

Figures 1A à 1D : Préparation des implants et procédure chirurgicale
Figure 2 : Illustration graphique de la concentration en calcium d'un plasma au contact des biomatériaux de phosphate de calcium.
Figure 3A : Photographie du produit obtenu par l'ajout d'une solution de chlorure de calcium au BCP et à l'HA avant l'addition de sang.
Figure 3B : Photographie du produit obtenu par l'ajout d'une solution de chlorure de calcium en concentrations croissantes sur du BCP.
Figure 4 : Analyse en microscopie électronique à balayage d'implants constitués de sang total prélevé sans anticoagulant (A, C) et de microparticules de BCP (80-200µm), ou d'implants préparés selon le protocole habituel (B, D). Dans les implants préparés sans calcium, on note l'absence de formation de réseau de fibrine et de caillot sanguin autour des grains (A, C). La flèche blanche en (C) indique quelques globules rouges déposés sur les grains. Echelles A, B: 100 µm, C, D : 10 µm.

### PARTIE EXPÉRIMENTALE

### I- Effet du BCP et de l'HA sur la coagulation :

### 1. Principe :

Il s'agit d'une procédure extemporanée, réalisée au bloc opératoire. Elle consiste à mélanger dans le corps d'une seringue en polypropylène des particules de BCP et un agent coagulant : CaCl₂. L'implantation de ce biomatériau dans le site où un défaut osseux a été constaté favorise la coagulation autour du biomatériau.

### 2. Matériels et Méthodes

### 2.1. Particules de phosphate de calcium biphasé :

Le biomatériau de phosphate de calcium biphasé (BCP) est composé de 60 % d'hydroxyapatite (HA; Ca₁₀(PO₄)₆(C)H)₂) et de 40% de phosphate tricalcique (TCP; Ca₃(PO₄)₂). Les particules de BCP calibrées entre 40 et 200 microns ont été fournies par la société GRAFTYS SARL (Aix-en-Provence, France). Les particules ont été stérilisées par chauffage à 180°C pendant deux heures.

### 2.2.Mesure de la concentration en calcium dans le plasma murin :

La concentration en calcium a été mesurée dans du plasma de souris C57BL/6 (Janvier, Le Genest-St-Isle, France). Ce plasma a été préparé à partir de sang prélevé sur héparine, par centrifugation à 1800g pendant 15 minutes. L'héparine est utilisée comme anticoagulant ne modifiant pas la concentration en calcium du plasma. L'analyse a été réalisée dans un automate Hitachi (Orléans, France).

### 2.3. Préparation des implants et procédure chirurgicale :

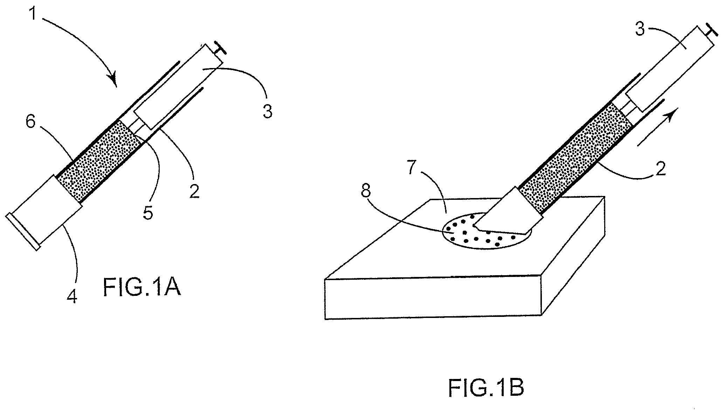

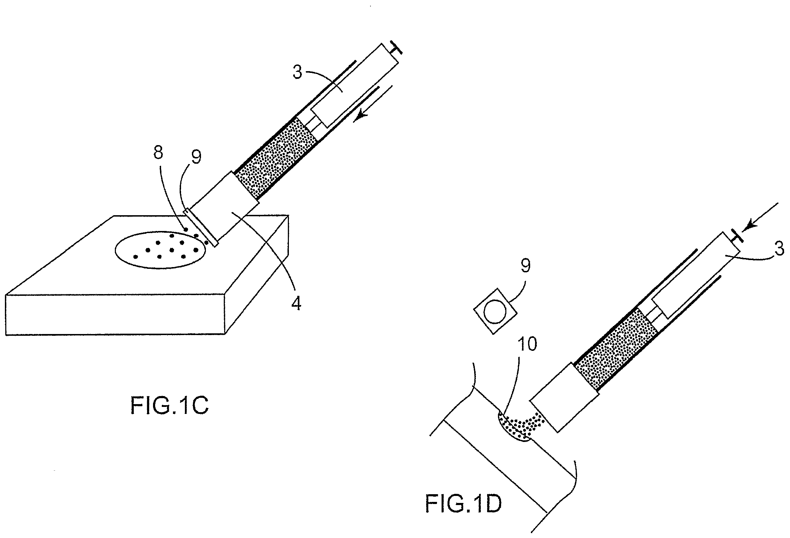

Comme illustré sur les figures 1A à 1D, on utilise une seringue (1) comportant un corps (2) cylindrique creux dans lequel se déplace un piston (3). A l'extrémité du corps (2) qui n'est pas obturée par le piston, le corps de la seringue est obturé par un bouchon filtre (4). Dans le corps (2) de la seringue, entre l'extrémité (5) du piston et le bouchon filtre (4) se trouvent des granules (6) de BCP (Figure 1A). L'ensemble a été stérilisé préalablement à son emploi. L'extrémité de la seringue portant le bouchon filtre (4) est placée dans un récipient (7) rempli d'une solution (8) aqueuse de CaCl₂ à une concentration de 1%. Un mouvement arrière du piston (3) permet d'aspirer la solution (8) à l'intérieur du corps (2) de la seringue (1) (Figure 1B). L'ensemble est laissé au repos pendant 10 mn afin que les particules de BCP s'imprègnent de la solution, puis à l'aide du piston (3) la solution (8) de CaCl₂ en excès est expulsée au travers du bouchon filtre (4) (Figure 1C). Le filtre (9) est retiré du bouchon-filtre (4) et une pression sur le piston (3) permet de déposer les granulés (6) de BCP imprégnés de solution (8) sur le site opératoire (10) (Figure 1D). Le site d'implantation est ensuite refermé (étape non représentée).

### 3. Résultats

### 3.1. Effet de l'hydroxyapatite et du TCP sur la coagulation :

50 mg de poudre de HA ou de poudre de TCP ont été placés dans le corps d'une seringue de 1 ml. 100 µl de sang ont été ajoutés dans chaque seringue contenant soit de l'HA, soit du TCP. Ce mélange a été placé sur une roue permettant de maintenir la poudre en suspension dans le sang pendant le temps de la coagulation, i.e 10 minutes. Dans chaque expérience une seringue contenant 100 µl de sang total traitée comme les autres i.e 10 min sur la roue, a servi de contrôle positif de la coagulation. Après 10 minutes, la roue est arrêtée, les seringues sont récupérées, leur bout est sectionné et le mélange sang/poudre est extrait en poussant avec le piston de la seringue. On observe ou pas la coagulation du sang autour de la poudre. Chaque expérience a été répétée 3 fois.

On a observé qu'en présence de 50 mg de HA et 100 µl de sang total la coagulation a été inhibée. Le sang reste liquide.

Témoin : contrôle positif de coagulation. On observe un caillot et un exsudat de sérum.

En présence de 50 mg de TCP + 100 µl de sang, la coagulation a eu lieu, elle se traduit par la formation d'un implant dans lequel le réseau de fibrine maintient la poudre en suspension de façon homogène.

La même expérience a été réalisée en ayant ajouté du chlorure de calcium dans la seringue contenant le HA préalablement à l'introduction de sang : on retrouve une coagulation et la formation d'un implant.

### 3.2. Effet du BCP sur la coagulation.

Nous avons observé que le sang fraîchement prélevé (100 µl) en absence d'anticoagulant et immédiatement mélangé aux particules de BCP (50 mg), ne coagule pas. Cet effet anticoagulant est annulé par l'ajout de CaCl₂ (20 µl d'une solution de CaCl₂ à 1%) suggérant une captation du calcium plasmatique par le BCP. Cette hypothèse a été confirmée par la mesure de la concentration en calcium dans le plasma avant et après contact avec du BCP. Pour cela, du plasma a été préparé à partir de sang de souris C57BL/6 prélevé sur héparine (anticoagulant ne modifiant pas la concentration plasmatique en calcium). En présence de BCP nous avons observé une chute de la concentration du calcium plasmatique de 2,06 ± 0,06 mmol / L (valeur normale) à 0,59 ± 0,07 mmol / L en présence de BCP.

### II- Effet des biomatériaux à base de phosphates de calcium sur la concentration du calcium plasmatique

### 1. Principe:

Des aliquots de 50 mg de microparticules de BCP (60/40), des aliquots de 50 mg de HA ou de β-TCP ont été mis en présence soit de 50 µl d'H₂O soit de 50 µl d'une solution à 2,5 mM de CaCl₂, 2H₂O, et mis à sécher pendant une nuit à 56°C. Ces biomatériaux ont été déposés dans les puits d'une microplaque de 96 puits. Dans chaque puits a été ajouté 100 µl de plasma préparé à partir de sang de souris C57BL/6 prélevé sur héparine, anticoagulant qui n'interfère pas avec le taux de calcium. Après 15 minutes d'incubation, la plaque a été centrifugée pendant 2 minutes à 800 g et les surnageants prélevés afin de doser la concentration en calcium du plasma. Le dosage du calcium a été réalisé en utilisant le Kit QuantiChrom Calcium Assay (CENTAUR, Bruxelles, Belgique) et en suivant les instructions du fabricant. Pour cela des aliquots de 5 µl de surnageant ont été mis en présence de 200 µl d'une solution de phenolsulphonephthalein, colorant qui forme un complexe stable de couleur bleue en présence de calcium libre. Après incubation pendant 3 minutes, on obtient une intensité de coloration mesurée à 612 nm, qui est directement proportionnelle à la concentration en calcium de l'échantillon. Dans chaque plaque, une gamme étalon est réalisée à partir des concentrations suivantes en calcium 0 - 0,5 - 1 - 1,5 - 2 - 3 - 4 - 5 mM.

### 2. Résultats :

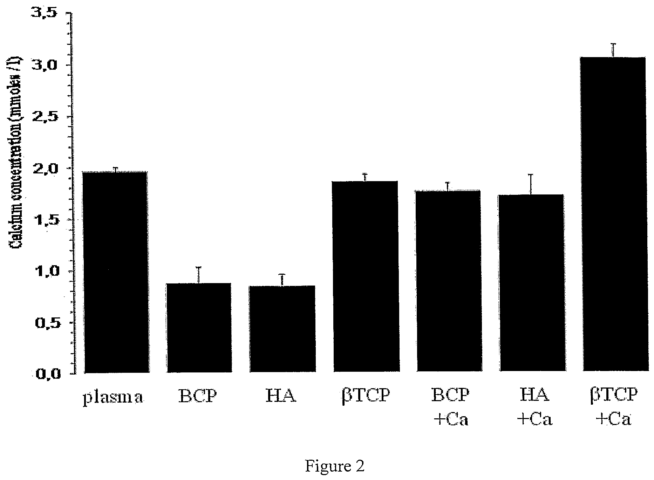

Nous avons constaté (Figure 2) que le BCP sous forme de microparticules ainsi que la poudre de HA, mis en contact avec du plasma, induisaient une diminution importante et significative de sa concentration en calcium. La chute de la concentration en calcium est similaire pour le BCP et l'HA et n'est pas observée pour le β-TCP. A partir des valeurs obtenues pour le plasma seul (1,960 ± 0,044 mM), le plasma en présence de BCP (0,871 ± 0,160 mM) et le plasma en présence de HA (0,840 ± 0,121 mM), nous avons évalué la captation de calcium à 0,125 µmole de calcium pour 50 mg de BCP ou de HA.

Nous avons également constaté que l'ajout de 50 µl d'une solution à 2,5 mM (soit 0,125 µmole) au BCP ou à l'HA avant d'ajouter le plasma permettait de restaurer une concentration plasmatique de calcium normale (Figure 2). La même quantité de chlorure de calcium ajoutée au β-TCP s'ajoute au calcium plasmatique initial et confirme l'absence de captation par ce biomatériau dans ces conditions.

Par ailleurs nous avons observé que la captation de calcium était identique pour les trois granulosités de BCP testées, c'est-à-dire pour les microparticules de 40-80 µm, de 80-200 µm et celles de 200-500 µm.

De plus nous avons obtenu les mêmes résultats de compensation par l'ajout de calcium, que la solution de chlorure de calcium soit ajoutée extemporanément sous forme liquide juste avant d'ajouter le plasma ou que cette solution soit d'abord séchée au contact des particules.

### III- Effet de l'ajout de calcium sur les propriétés anticoagulantes du BCP et de l'HA

### 1. Principe:

Afin de démontrer qu'il existait un lien de cause à effet entre l'inhibition de la coagulation et la chute du calcium plasmatique induite par le BCP et l'HA, nous avons réalisé des essais de coagulation sur des aliquots de 50 mg de microparticules de BCP (60/40) et de 50 mg de poudre de HA, après ajout de 50 µl de 150 mM NaCl ou de 50 µl de 2,5 mM CaCl₂, 2H₂O.

### 2. Résultats:

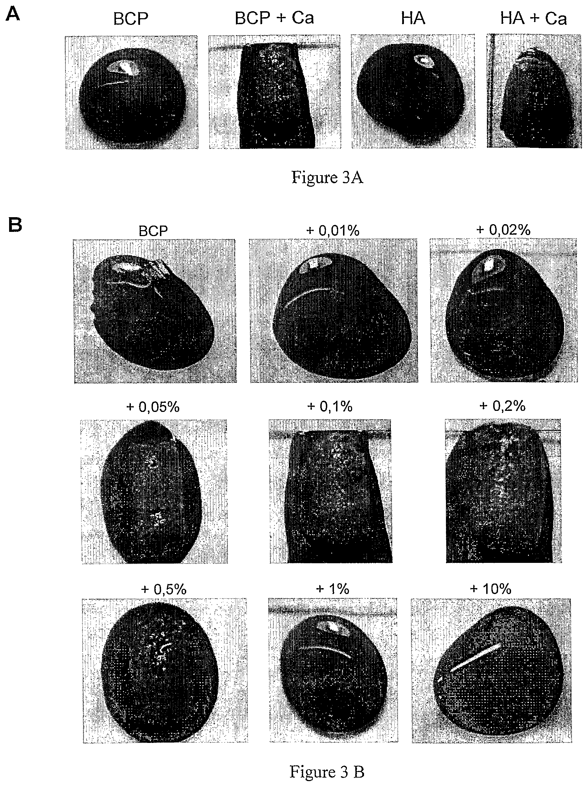

Après ajout du sang non traité par un anticoagulant et rotation pendant 15 minutes nous avons constaté (Figure 3A) que l'ajout préalable de calcium au BCP et à l'HA permettait de rétablir la coagulation du sang mis en présence de ces deux biomatériaux.

Ces résultats démontrent que l'effet anticoagulant du BCP et de l'HA est bien lié à la diminution de la concentration en calcium plasmatique que ces deux biomatériaux induisent, et que l'ajout de calcium permet de rétablir la coagulation.

Nous avons analysé l'effet d'une dose réponse de calcium sur la coagulation du sang mis en contact avec le BCP (Figure 3B). Le biomatériau a été préparé en ajoutant 100 µl de sang total non additionné d'un anticoagulant à 50 mg de particules de BCP en présence d'un volume fixe de 50 µl de CaCl₂,2H₂O, préparé aux concentrations de 0,01 % (0,68 mM) - 0,02 % (1,36mM) - 0,05 % (3,4 mM) - 0,1 % (6,8 mM) - 0,2 % (13,6 mM) - 0,5 % (34 mM) - 1 % (68 mM) - 10% (680 mM) ou du même volume de NaCl à 150 mM. Après incubation pendant 15 minutes sur une roue, le biomatériau est démoulé. Nous avons constaté qu'à faible concentration, correspondant ici à 0,01 et 0,02 %, le calcium ajouté ne permettait pas de rétablir la coagulation. En présence de concentrations comprises entre 0,05 et 0,5 % on observe une coagulation. De façon surprenante, nous avons constaté que l'augmentation de la concentration en CaCl₂, 2H₂O à 1% et au-delà induisaient à nouveau une inhibition de la coagulation (Figure 3B et tableau 1).

Ces expériences ont permis de déterminer les concentrations optimales en calcium permettant de bloquer l'effet anticoagulant du BCP 60/40 et ont montré qu'il existe une fourchette de concentration très importante à respecter.

### IV. Analyse du réseau de fibrine en microscopie électronique à balayage

L'effet anticoagulant du BCP visualisé par l'absence de formation d'implants gélifiés cohésifs au cours des essais décrits ci-dessus, correspond au niveau moléculaire à l'inhibition de la formation du réseau de fibrine formant l'armature du caillot. Nous avons analysé la présence du réseau fibrine en microscopie électronique à balayage. Pour cela des implants ont été préparés en mélangeant 100 µl de sang non additionné d'anticoagulant avec 50 mg de BCP avec ou 50 mg de BCP incubé en présence de calcium puis séché. Après 15 minutes de rotation sur une roue, les mélanges sont démoulés et directement plongés dans une solution de fixation à 1,6% de glutaraldéhyde dans un tampon phosphate 0,1M pH 7. Les échantillons sont ensuite lavés, déshydratés par des bains d'alcool à concentrations croissantes, immergés en hexamethyldisilazane (Sigma-Aldrich, L'isle d'Abeau Chesnes, France) pendant 5 minutes et séchés à température ambiante. Après montage sur des supports en aluminium et recouvrement à l'or palladium pendant 4 minutes (Polaron, A5100, UK), l'analyse est réalisée à l'aide d'un microscope électronique à balayage (JEOL 6700F, Japan).

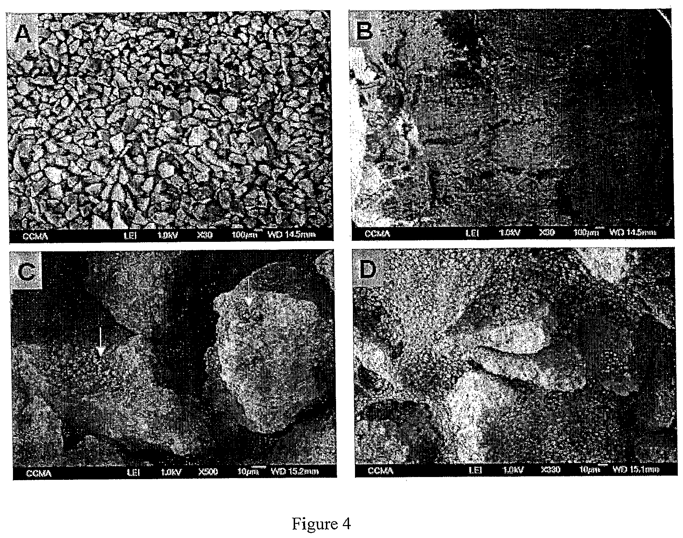

Comme on le voit sur la figure 4, dans les conditions BCP nous n'avons pas observé de réseau de fibrine (Figures 4A, 4C) entre les microparticules de BCP. La présence de quelques globules rouges déposés sur les grains atteste du mélange des particules avec le sang. Au contraire, en présence de BCP/calcium on constate la présence d'un caillot enserrant les particules, caillot visualisé par les mailles du réseau de fibrine et un très grand nombre de globules rouges (Figures 4B, 4D).

**Tableau 1**

| Concentration de la solution de CaCl₂,2H₂O ajoutée au mélange de BCP et de sang (en % et en molarité) | Nombre de µmoles de calcium apportées par 50 µl de CaCl₂, 2H₂O | Test de coagulation 50 mg BCP + 100 µl sang + 50µl CaCl₂, 2H₂O (liq ou séché) |
|---|---|---|
| 0 | | - |
| 0,01% (0,68 mM) | 0,034 | - |
| 0,02 % | 0,068 | + |
| 0,03 % | 0,102 | + |
| 0,04 % | 0,136 | + |
| 0,05 % | 0,17 | + |
| 0,1 % | 0,34 | + |
| 0,2 % | 0,68 | + |
| 0,5 % | 1,7 | + |
| 0,6 % | 2,04 | + |
| 0,7 % | 2,38 | + |
| 0,8 % | 2,72 | + |
| 0,9 % | 3,06 | + |
| 1 % (68 mM) | 3,4 | +/- |
| 2% | | - |
| 10 % (680 mM) | 34 | - |

## Revendications

1. Procédé de préparation d'un biomatériau comprenant un support à base de phosphate de calcium, **caractérisé en ce que** :
- le support est choisi parmi l'hydroxyapatite et un BCP, de préférence un BCP, et
- **en ce que** ledit support est imprégné par au moins un agent coagulant dérivé du calcium, de préférence CaCl₂, sous forme d'une solution aqueuse d'une concentration allant de 1 à 50 mMol/l, de préférence de 3 à 40 mMol/l, avantageusement de 5 à 20 mMol/l,
la proportion de solution d'agent coagulant et d'hydroxyapatite ou de BCP étant de 0,5 à 5, de préférence de 1 à 3, en volume de solution d'agent coagulant par rapport au volume d'hydroxyapatite ou de BCP.

2. Biomatériau comprenant un support à base de phosphate de calcium, imprégné par une solution d'au moins un agent coagulant dérivé du calcium, **caractérisé en ce que** le support est choisi parmi l'hydroxyapatite et un BCP, et **en ce que** l'agent coagulant dérivé du calcium est présent en une proportion allant de 2,5 à 60 µmol de calcium par gramme d'hydroxyapatite ou de BCP, et de préférence de 5 à 40 µmol de calcium par gramme d'hydroxyapatite ou de BCP.

3. Biomatériau comprenant un support à base de phosphate de calcium, associé à au moins un agent coagulant dérivé du calcium sous forme de poudre, **caractérisé en ce que** le support est choisi parmi l'hydroxyapatite et un BCP, et **en ce que** l'agent coagulant dérivé du calcium est présent en une proportion allant de 2,5 à 60 µmol de calcium par gramme d'hydroxyapatite ou de BCP, et de préférence de 5 à 40 µmol de calcium par gramme d'hydroxyapatite ou de BCP.

4. Biomatériau selon la revendication 2 ou selon la revendication 3, dans lequel l'agent coagulant est CaCl₂.

5. Biomatériau selon l'une quelconque des revendications 2 à 4 dans lequel le support est sous forme de monolithe.

6. Biomatériau selon l'une quelconque des revendications 2 à 4 dans lequel le support est sous forme de granulés.

7. Biomatériau selon l'une quelconque des revendications 2 à 6 et comprenant en outre au moins un additif choisi parmi : des polymères, des particules de céramiques, des molécules pharmaceutiques, des facteurs de croissance, naturels ou de synthèse, des biomarqueurs, des agents de contraste, une préparation de tissu ou de cellules.

8. Biomatériau selon l'une quelconque des revendications 2 à 7 ou obtenu selon la revendication 1 pour son utilisation comme implant dans un procédé de comblement d'un défaut osseux.

9. Association d'un biomatériau selon l'une quelconque des revendications 2 à 7 ou obtenu selon le procédé de la revendication 1 avec une ostéosynthèse.

10. Kit pour la mise en oeuvre du procédé de fabrication d'un biomatériau selon l'une quelconque des revendications 2 et 4 à 7 ou obtenu selon le procédé de la revendication 1, ce kit comprenant
- un support à base d'hydroxyapatite ou de BCP, et
- un agent coagulant dérivé du calcium, sous forme d'une solution aqueuse de concentration allant de 1 à 50 mM/l et la proportion de solution d'agent coagulant et d'hydroxyapatite ou de BCP est de 0,5 à 5 en volume/volume de solution d'agent coagulant par rapport au volume d'hydroxyapatite ou de BCP.

11. Kit pour la mise en oeuvre d'un procédé de fabrication d'un biomatériau selon l'une quelconque des revendications 3 à 7, ce kit comprenant
- un support à base d'hydroxyapatite ou de BCP, et
- un agent coagulant dérivé du calcium sous forme de poudre.

12. Kit selon la revendication 10 ou 11 qui comporte :
(a) un dispositif stérile comportant une cavité intérieure stérile dans lequel est placé le support,
(b) un réservoir stérile comportant l'agent coagulant.

13. Kit selon la revendication 12 dans lequel le dispositif (a) comporte des moyens permettant l'application du biomatériau dans la zone où un défaut osseux a été constaté.

14. Kit selon la revendications 12 ou 13 dans lequel le dispositif (a) est constitué d'une seringue.

15. Utilisation d'un biomatériau selon l'une quelconque des revendications 2 à 7 ou obtenu selon le procédé de la revendication 1, *in vitro* ou *ex*-*vivo* comme support pour la production de tissu osseux, ou pour produire un implant osseux.

## Patentansprüche

1. Verfahren zur Herstellung eines Biomaterials umfassend einen Träger auf Calciumphosphatbasis, **dadurch gekennzeichnet, dass**:
- der Träger aus Hydroxyapatit und einem BCP, vorzugsweise einem BCP, ausgewählt ist und
- dadurch, dass der Träger mit mindestens einem Koagulationsmittel, das sich von Calcium ableitet, vorzugsweise CaCl₂, in Form einer wässrigen Lösung mit einer Konzentration von 1 bis 50 mMol/l, vorzugsweise 3 bis 40 mMol/l, vorteilhafterweise 5 bis 20 mMol/l imprägniert ist,
wobei das Verhältnis von Koagulationsmittellösung und Hydroxyapatit oder BCP 0,5 bis 5, vorzugsweise 1 bis 3 in Bezug auf das Volumen der Koagulationsmittellösung in Bezug auf das Hydroxyapatit- oder BCP-Volumen beträgt.

2. Biomaterial umfassend einen Träger auf Calciumphosphatbasis, der mit einer Lösung von mindestens einem Koagulationsmittel, das sich von Calcium ableitet, imprägniert ist, **dadurch gekennzeichnet, dass** der Träger aus Hydroxyapatit und einem BCP ausgewählt ist und dadurch, dass das Koagulationsmittel, das sich von Calcium ableitet, in einem Verhältnis von 2,5 bis 60 µmol Calcium pro Gramm Hydroxyapatit oder BCP, vorzugsweise 5 bis 40 µmol Calcium pro Gramm Hydroxyapatit oder BCP, vorliegt.

3. Biomaterial umfassend einen Träger auf Calciumphosphatbasis in Kombination mit mindestens einem Koagulationsmittel, das sich von Calcium ableitet, in Form eines Pulvers, **dadurch gekennzeichnet, dass** der Träger aus Hydroxyapatit und einem BCP ausgewählt ist, und dadurch, dass das Koagulationsmittel, das sich von Calcium ableitet, in einem Verhältnis von 2,5 bis 60 µmol Calcium pro Gramm Hydroxyapatit oder BCP, vorzugsweise 5 bis 40 µmol Calcium pro Gramm Hydroxyapatit oder BCP, vorliegt.

4. Biomaterial nach Anspruch 2 oder nach Anspruch 3, wobei es sich bei dem Koagulationsmittel um CaCl₂ handelt.

5. Biomaterial nach einem der Ansprüche 2 bis 4, wobei der Träger in Monolithform vorliegt.

6. Biomaterial nach einem der Ansprüche 2 bis 4, wobei der Träger in Granulatform vorliegt.

7. Biomaterial nach einem der Ansprüche 2 bis 6, das weiterhin mindestens einen Zusatzstoff, ausgewählt aus dem Folgenden, umfasst: Polymeren, Keramikteilchen, pharmazeutischen Molekülen, natürlichen oder synthetischen Wachstumsfaktoren, Biomarkern, Kontrastmitteln, einem Gewebepräparat oder Zellen.

8. Biomaterial nach einem der Ansprüche 2 bis 7 oder erhalten nach Anspruch 1 zur Verwendung als Implantat in einem Verfahren zum Auffüllen eines Knochendefekts.

9. Kombination eines Biomaterials nach einem der Ansprüche 2 bis 7 oder erhalten nach dem Verfahren von Anspruch 1 mit einer Osteosynthese.

10. Kit zur Durchführung des Verfahrens zur Erzeugung eines Biomaterials nach einem der Ansprüche 2 und 4 bis 7 oder erhalten nach dem Verfahren von Anspruch 1, wobei das Kit Folgendes umfasst:
- einen Träger auf Hydroxyapatit- oder BCP-Grundlage und
- ein Koagulationsmittel, das sich von Calcium ableitet, in Form einer wässrigen Lösung mit einer Konzentration von 1 bis 50 mMol/l und das Verhältnis der Lösung des Koagulationsmittels und des Hydroxyapatits oder BCPs beträgt 0,5 bis 5 Volumen/Volumen Koagulationsmittellösung im Vergleich zum Hydroxyapatit- oder BCP-Volumen.

11. Kit zur Durchführung des Verfahrens zur Erzeugung eines Biomaterials nach einem der Ansprüche 3 bis 7, wobei das Kit Folgendes umfasst
- einen Träger auf Hydroxyapatit- oder BCP-Grundlage und
- ein Koagulationsmittel, das sich von Calcium ableitet, in Pulverform.

12. Kit nach Anspruch 10 oder 11, das Folgendes umfasst:
(a) eine sterile Vorrichtung, die eine sterile innere Ausnehmung umfasst, in die der Träger platziert wird,
(b) ein steriles Behältnis, das das Koagulationsmittel umfasst.

13. Kit nach Anspruch 12, wobei die Vorrichtung (a) Mittel umfasst, die die Applikation des Biomaterials dorthin, wo ein Knochendefekt diagnostiziert wurde, gestattet.

14. Kit nach Anspruch 12 oder 13, wobei die Vorrichtung (a) aus einer Spritze besteht.

15. In-vitro- oder ex-vivo-Verwendung eines Biomaterials nach einem der Ansprüche 2 bis 7 oder erhalten nach dem Verfahren von Anspruch 1 als Träger für die Produktion von Knochengewebe oder zur Herstellung eines Knochenimplantats.

## Claims

1. Method for preparing a biomaterial comprising a support containing calcium phosphate, **characterized in that**:
- the support is chosen from hydroxyapatite and a BCP, preferably a BCP, and
- **in that** said support is impregnated with at least one calcium-based coagulant, preferably CaCl₂, in the form of an aqueous solution with a concentration ranging from 1 to 50 mMol/l, preferably from 3 to 40 mMol/l, advantageously from 5 to 20 mMol/l, the proportion of solution of coagulant and of hydroxyapatite or of BCP being from 0.5 to 5, preferably from 1 to 3, by volume of coagulant solution relative to the volume of hydroxyapatite or of BCP.

2. Biomaterial comprising a support containing calcium phosphate, impregnated with a solution of at least one calcium-based coagulant, **characterized in that** the support is chosen from hydroxyapatite and a BCP, and **in that** the calcium-based coagulant is present in a proportion ranging from 2.5 to 60 µmol of calcium per gram of hydroxyapatite or of BCP, and preferably from 5 to 40 µmol of calcium per gram of hydroxyapatite or of BCP.

3. Biomaterial comprising a support containing calcium phosphate, combined with at least one calcium-based coagulant in powder form, **characterized in that** the support is chosen from hydroxyapatite and a BCP, and **in that** the calcium-based coagulant is present in a proportion ranging from 2.5 to 60 µmol of calcium per gram of hydroxyapatite or of BCP, and preferably from 5 to 40 µmol of calcium per gram of hydroxyapatite or of BCP.

4. Biomaterial according to Claim 2 or according to Claim 3, in which the coagulant is CaCl₂.

5. Biomaterial according to any one of Claims 2 to 4, in which the support is in monolith form.

6. Biomaterial according to any one of Claims 2 to 4, in which the support is in granule form.

7. Biomaterial according to any one of Claims 2 to 6 and also comprising at least one additive chosen from: polymers, ceramic particles, pharmaceutical molecules, natural or synthetic growth factors, biomarkers, contrast agents, a tissue or cell preparation.

8. Biomaterial according to any one of Claims 2 to 7 or obtained according to Claim 1, for use thereof as an implant in a method for filling a bone defect.

9. Combination of a biomaterial according to any one of Claims 2 to 7 or obtained according to the method of Claim 1, with an osteosynthesis.

10. Kit for carrying out the method for producing a biomaterial according to any one of Claims 2 and 4 to 7 or obtained according to the method of Claim 1, this kit comprising
- a support containing hydroxyapatite or BCP, and
- a calcium-based coagulant, in the form of an aqueous solution with a concentration ranging from 1 to 50 mM/l and the proportion of solution of coagulant and of hydroxyapatite or of BCP being from 0.5 to 5 by volume/volume of coagulant solution relative to the volume of hydroxyapatite or of BCP.

11. Kit for carrying out a method for producing a biomaterial according to any one of Claims 3 to 7, this kit comprising
- a support containing hydroxyapatite or BCP, and
- a calcium-based coagulant in powder form.

12. Kit according to Claim 10 or 11, which comprises:
(a) a sterile device comprising a sterile interior cavity in which the support is placed,
(b) a sterile reservoir comprising the coagulant.

13. Kit according to Claim 12, in which the device (a) comprises means for applying the biomaterial in the area where a bone defect has been noted.

14. Kit according to Claim 12 or 13, in which the device (a) consists of a syringe.

15. Use of a biomaterial according to any one of Claims 2 to 7 or obtained according to the method of Claim 1, *in vitro* or *ex vivo* as a support for the production of bone tissue, or for producing a bone implant.
